# EUROPEAN PATENT SPECIFICATION

(11) **EP 3 877 038 B1**
(45) Date of publication and mention of the grant of the patent: **14.08.2024**
(21) Application number: 19882624.0
(22) Date of filing: 06.11.2019
(51) Int. Cl.: A61N 1/05

(54) **APPARATUS FOR A LEAD FIXATION DEVICE**
EINRICHTUNG FÜR EINE LEITUNGSBEFESTIGUNGSVORRICHTUNG
APPAREIL POUR UN DISPOSITIF DE FIXATION D'UN CONDUCTEUR ÉLECTRIQUE

(30) Priority: 06.11.2018 US 201862756185 P; 07.12.2018 US 201862776550 P
(43) Date of publication of application: 15.09.2021
(73) Proprietor: Smith, Kris A., Phoenix, AZ 85022 (US)
(72) Inventor: MAREK, Matthew, G., Plano, TX 75023 (US); SMITH, Kris, A., Phoenix, AZ 85022 (US)
(74) Representative: K&L Gates LLP
(86) International application number: PCT/US2019/059995
(87) International publication number: WO 2020/097159

(56) References cited:
- EP-B1- 1 048 320
- US-A- 5 927 277
- US-A- 5 927 277
- US-A- 6 134 477
- US-A1- 2005 192 594
- US-A1- 2013 197 472
- US-A1- 2014 276 418
- US-A1- 2015 039 063

## Description

### BACKGROUND OF THE TECHNOLOGY

Deep Brain Stimulation (DBS) is an established neurosurgical treatment for multiple conditions including: Parkinson's disease, essential tremor, dystonia, and has been recently approved for medically refractory epilepsy. Responsive neural stimulation also uses implanted electrodes in the brain and is FDA approved. Additional probable indications likely to be approved in the future include: obsessive compulsive disorder, depression and even Alzheimer's disease. The number of permanently implanted brain electrodes continues to increase each year. Each brain electrode requires precise placement and permanent fixation, which allows for years of use. Generally the brain electrodes consist of DBS leads, which are implanted into the brain. The DBS leads are designed for durability and precise control of therapy.

DBS surgery typically involves placement of a single lead through a burr hole or a single twist drill opening, either unilaterally or bilaterally. A burr hole is a relatively small opening made in the bone of the skull to access the intracranial space (the space inside the skull). Burr holes are used to help relieve pressure on the brain when fluid, such as blood, builds up and starts to compress brain tissue. A burr hole is typically made by some form of spinning burr tool which can either be a manual, electric or pneumatic drill. The cutting bit is very much like a drill bit and allows the neurosurgeon to drill through the skull in a controlled fashion. The size of a typical burr hole is 14 mm. The size of a single twist drill opening may range from 2-5mm in diameter for a single lead position. Other sizes may be contemplated based on the size of the lead fixation device.

In practice a lead is stereotactically implanted through a separate burr hole or single twist drill opening and then secured in place with a lead fixation device. A standard lead fixation device anchors the lead to the skull and covers the burr hole or single twist drill opening drilled through the skull. Standard lead fixation devices are designed for stable and secure fit against the skull and burr hole or single twist drill opening and to protect the lead/clip interface and create a smooth profile over the burr hole or single twist drill opening

Unfortunately, all currently available lead fixation devices are somewhat cumbersome to use and result in a visible and palpable protrusion under the skin, which is usually bothersome to the patient. The visibility of the current lead fixation devices may also limit the use of DBS leads with bald patients, who may be concerned about the appearance of "horns." One such example is the SureTek^{™} Burr Hole Cover Kit provided by Boston Scientific^{®}.

US 6134477A discloses an adjustable medical apparatus for anchoring a lead to a cranial burr hole. The apparatus comprises generally an anchoring plate mounted to and over a cranial burr hole, an anchoring arm pivotally mounted to the anchoring plate, and a resilient sleeve mounted between the anchoring plate and the anchoring arm. The resilient sleeves defines an aperture for receiving the lead. Once an implanted lead is inserted into the aperture in the resilient sleeve, the anchoring arm is pivoted around the sleeve creating a clamping force on the resilient sleeve. The resilient sleeve in turn creates a clamping force on the lead body, thereby anchoring the lead relative to the anchoring apparatus and thus relative to the cranium. Significantly, the anchoring apparatus accommodates various sized lead bodies which are used within various sized burr holes.

US 5927277A discloses an apparatus and method for securing medical devices such as intracerebroventricular and parenchymal catheters and electrical stimulation leads within a cranial burr hole comprising a snap ring assembly selectively secured at the burr hole so that an extension of the assembly, having one or more points of fixation for a catheter or lead, secures a catheter or lead extending through the burr hole at a desired point, thereby minimizing undesirable movement of the positioned medical device. The catheter or lead is secured by opposed edges of bar extensions that are placed about the lead or catheter when the snap ring assembly is squeezed and a space between the edges is opened, but that close upon and hold the catheter or lead when the snap ring is released; by a scissors-like jaws mechanism operatively coupled to the snap ring assembly which clamps and holds the positioned device; or by a rigid extension having at least one fixation point through which the catheter or lead passes and at which the catheter or lead is secured

### SUMMARY OF THE TECHNOLOGY

The claimed invention is disclosed in claim 1, to which reference should now be made Various optional features are disclosed in the dependent claims.

### BRIEF DESCRIPTION OF THE DRAWINGS

A more complete understanding of the present technology may be derived by referring to the detailed description when considered in connection with the following illustrative figures. In the following figures, like reference numbers refer to similar elements and steps throughout the figures.
Figure 1 representatively illustrates a top view of a lead fixation device in a closed position in accordance with various embodiments of the present technology;
Figure 2 representatively illustrates a top view of the lead fixation device in an open position in accordance with various embodiments of the present technology;
Figure 3 representatively illustrates an exploded, perspective view of the lead fixation device in an open position in accordance with various embodiments of the present technology
Figure 4 representatively illustrates a top view of an additional embodiment of a lead fixation device in an open position in accordance with various embodiments of the present technology;
Figure 5 representatively illustrates a side view of the additional embodiment of a lead fixation device in an open position in accordance with various embodiments of the present technology;
Figure 6 representatively illustrates a front side, perspective view of the additional embodiment of a lead fixation device in an open position in accordance with various embodiments of the present technology;
Figure 7 representatively illustrates a rear side, perspective view of the additional embodiment the lead fixation device in an open position in accordance with various embodiments of the present technology;
Figure 8 representatively illustrates a front side, perspective view the additional embodiment of the lead fixation device in an installed position in accordance with various embodiments of the present technology;
Figure 9 representatively illustrates a bottom view of an additional embodiment of a lead fixation device in an open position in accordance with various embodiments of the present technology;
Figure 10 representatively illustrates a top view of the additional embodiment of the lead fixation device in an open position in accordance with various embodiments of the present technology; and
Figure 11 representatively illustrates a top view of the additional embodiment of the lead fixation device in a closed position in accordance with various embodiments of the present technology.

Elements and steps in the figures are illustrated for simplicity and clarity and have not necessarily been rendered according to any particular sequence. For example, steps that may be performed concurrently or in a different order are illustrated in the figures to help to improve understanding of embodiments of the present technology.

### DETAILED DESCRIPTION OF EXEMPLARY EMBODIMENTS

The present technology may be described in terms of functional block components and various processing steps. Such functional blocks may be realized by any number of components configured to perform the specified functions and achieve the various results. For example, the present technology may employ various types of materials, connectors, and the like for a lead fixation device, which may carry out a variety of operations suited to a specified application or environment. In addition, the present technology may be practiced in conjunction with any number of systems configured for operation with lead fixation devices, and the systems described are merely exemplary applications for the technology. Further, the present technology may employ any number of conventional techniques for utilizing and practicing lead fixation devices.

Methods and apparatus for providing a lead fixation device designed to minimize an invasive lead placement within the user and reduce visibility of the device to the public. Various representative implementations of the present technology may be applied to any system for minimizing invasive lead placement within the user and reduce visibility of the device to the public. The present technology may be utilized to minimize invasive lead placement within the user and reduce visibility of the device to the public.

The lead fixation devices described below may be comprised of any suitable materials. For example, the lead fixation device may comprise titanium, various plastic materials, or any other suitable medical grade material. In one embodiment, the lead fixation devices may be made of biocompatible Polyether ether ketone ("PEEK") material, which avoids wear and/or excessive compression of the lead. PEEK is a colourless organic thermoplastic polymer in the polyaryletherketone family, typically used in engineering applications. The PEEK material is constructed without sharp edges so that damage to the Deep Brain Stimulation ("DBS") lead does not occur.

Accordingly, a lead fixation device 100 is contemplated that is minimally invasive, smaller, and easier to install than any currently available system. The lead fixation device 100 allows the DBS lead to be placed through a small incision and bone opening than other current lead fixation devices. For example, the present lead fixation device 100 may be implanted in a bone opening of 3 mm. The smaller bone opening may be referred to as a twist drill hole or a biopsy drill hole. It should be understood that the lead fixation device 100 can be sized according to any suitable bone opening. In various embodiments, the lead fixation device 100 may be utilized with a single twist drill opening ranging from 2-5mm in diameter. The lead fixation device 100 does not require any assembly or removal of the stereotactic at the time of lead fixation. In various embodiments, the height of the lead fixation device 100 may be range approximately 1mm-3mm. In one embodiment, the height of the lead fixation device 100 may be approximately 2mm. In one embodiment, the height of the lead fixation device 100 may be approximately 1mm. In one embodiment, the height of the lead fixation device 100 may be less than 1mm.

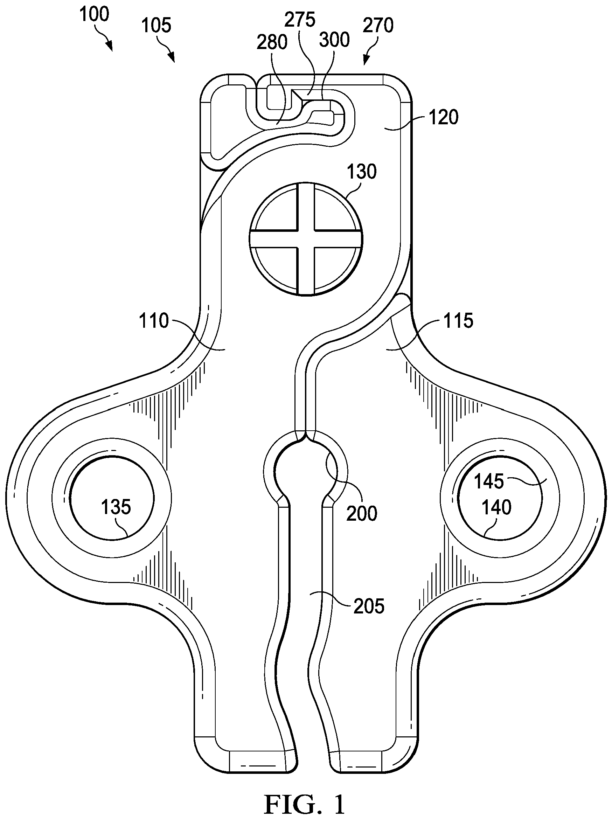

In accordance with various embodiments, referring now to Figures 1-3, the lead fixation device 100 may comprise a mounting plate 105 comprising a first arm 110 and a second arm 115. In one embodiment, the first and second arms 110, 115 may comprise first and second flanges 120, 125. The first flange 120 of the first arm 110 may be coupled to the second flange 125 of the second arm 115. In one embodiment, first and second flanges 120, 125 of the first and second arms 110, 115 are rotatable and coupled to each other. In one embodiment, first and second flanges 120, 125 of the first and second arms 110, 115 are press-fit together at the rotating hinge site with a circular flange collar. In other words one fitting inside in each arm resides within the other. In another embodiment, a coupling mechanism 130 is used to couple the first and second arms 110, 115 to one another. The coupling mechanism 130may be any screw, flange or coupling combination that couples the first and second arms 110, 115 to one another. The second arm 115 is rotatable with regard to the first arm 110 such that when the two arms are pressed together, the lead fixation device 100 may secure the DBS lead to the patient/user.

In various embodiments, the first and second arms 110, 115 of the mounting plate 105 may comprise a pair of mounting holes 135, 140, which extend therethrough. The first and second mounting holes 135, 140 may be generally cylindrical. The first and second mounting holes 135, 140 may be fixed once installed. Each of the first and second mounting holes 135, 140 may contain an inwardly beveled sidewall 145 that receives a countersunk head of a mounting screw (not shown). Each of the first and second mounting holes 135, 140 may be located at approximately the midpoint of the first and second arms 110, 115.

In one embodiment, the first and second arms 110, 115 of the mounting plate 105 may each comprise an inner wall 150, 155. The inner walls 150, 155 each may comprise a mating portion 160, 165, an arcuate groove 170, 175, and a longitudinal wall 180, 185. In one embodiment, the longitudinal walls 180, 185, may comprise a curved portion 190, 195.

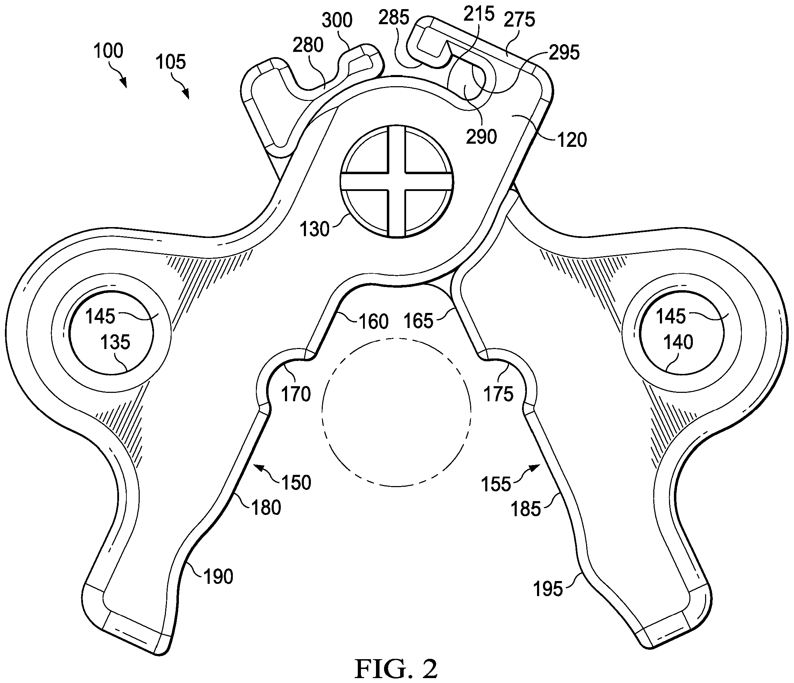

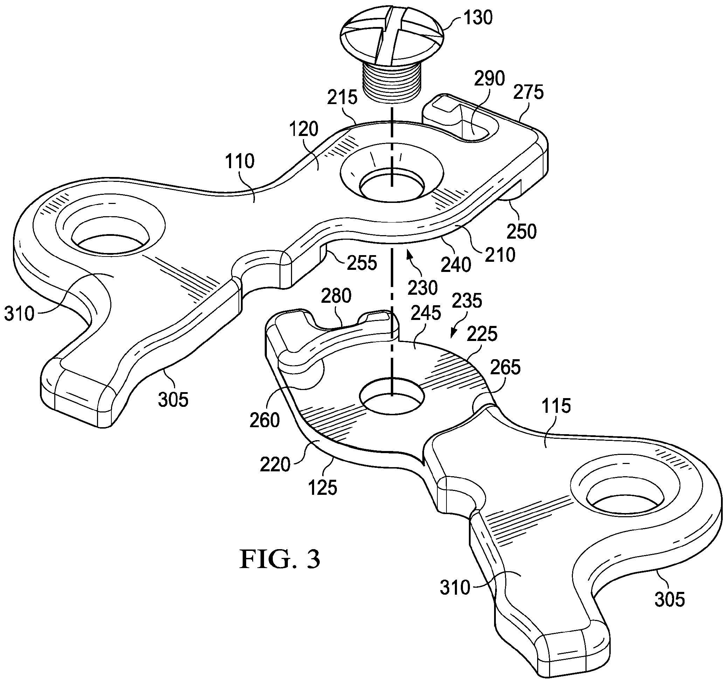

Figure 1 shows the lead fixation device 100 in closed position while Figures 2 and 3 show the lead fixation device 100 in an open position. In the closed position, mating portion 160, 165 abut and the arcuate groove 170, 175 form at least one guide channel 200 and the longitudinal walls 180, 185 form an exit channel 205. The guide channel 200 receives the DBS lead that exits the user's skull. In the closed position, the exit channel 205 formed within the space between the first and second arms 110, 115 guides the DBS lead that exits the user's skull in a desired direction. In one embodiment, the first and second mounting holes 135, 140 are located generally outwardly from the at least one guide channel 200. The curved portions 190, 195 and the longitudinal walls 180, 185 when combined in the closed position form a "slalom slot" for press fitting the lead in its final position, after the lead stylet is removed, and to secure the lead against any delayed migration or external damage at the bending point where the lead exits the skull via the guide channel 200.

Referring now to Figure 3, the first and second flanges 120, 125 will be discussed. In various embodiments, the first flange 120 may comprise first and second outer walls 210, 215 and the second flange may comprise first and second outer walls 220, 225. The outer walls 210, 215, 220, and 225 are generally curved and assist in the coupling and rotation of the first and second flanges 120, 125. In various embodiments, the first and second flanges 120, 125 may comprise first and second mating portions 230, 235. In various embodiments, each of the first and second mating portions may comprise recessed areas 240, 245 where a portion of the first and second flanges 120, 125 has been removed. In various embodiments, the first flange 120 may comprise first and second collar walls 250, 255 and the second flange 125 may comprise first and second collar walls 260, 265. The first and second collar walls 250, 255 of the first flange 120 are oriented opposite one another with the recessed area 240 locate there between. The first and second outer walls 210, 215 are located on the perimeter of the recessed area 240 between the first and second collar walls 250, 255. The first and second collar walls 260, 265 of the second flange 125 are oriented opposite one another with the recessed area 245 locate there between. The first and second outer walls 220, 225 are located on the perimeter of the recessed area 245 between the first and second collar walls 260, 265.

When assembled the recessed areas 240, 245 of the first and second mating portions 230, 235 contact one another; the outer walls 210, 215 of the first flange 120 are located adjacent the first and second collar walls 260, 265 of the second flange 125; and the outer walls 220, 225 of the second flange 125 are located adjacent the first and second collar walls 250, 255 of the first flange 120. This configuration allows the first arm 110 and second arm 115 to rotate with respect to each other and the height of the lead fixation device 100 to remain consistent.

In various embodiments, the lead fixation device 100 may comprise a locking mechanism 270. The locking mechanism 270 may comprise first and second fingers 275, 280. The first finger 275 depends from an upper surface of the first flange 120 of the first arm 110 and comprises a raised portion 285. The first finger 275 is oriented such that a space 290 is formed between a lower surface 295 of the first finger 275 and the outer wall 215. The second finger 280 depends from an upper surface of the second flange 125 of the second arm 115 and comprises a raised portion 300. When the lead fixation device 100 is oriented in the closed position of Figure 1, the raised portion 300 is received within the space 290 and the raised portions 285, 300 lock the first and second arms 110, 115 of the lead fixation device 100.

A lower surface 305 of the mounting plate 105 generally abuts the patient/user's skull while an upper surface 310 of the mounting plate 105 is generally flush with or sits on top of the patient/user's scalp. The lower surface 305 of the mounting plate 105 may be generally flat so that the lead fixation device is generally flush with the patient/user's scalp. This configuration provides a generally invisible or inconspicuous lead fixation device.

In operation, the DBS lead is inserted within a burr hole or a single twist drill opening in the user/patient's skull. While the lead fixation device 100 in oriented in the open position, shown in Figure 2, the DBS lead is placed within the guide channel 200. The doctor may then press or pinch the arms 115, 120 toward one another and insert screws within the pair of mounting holes 135, 140 to fasten the lead fixation device 100 to the skull. The DBS lead is then placed within the exit channel 205 formed within the space between the first and second arms 110, 115.

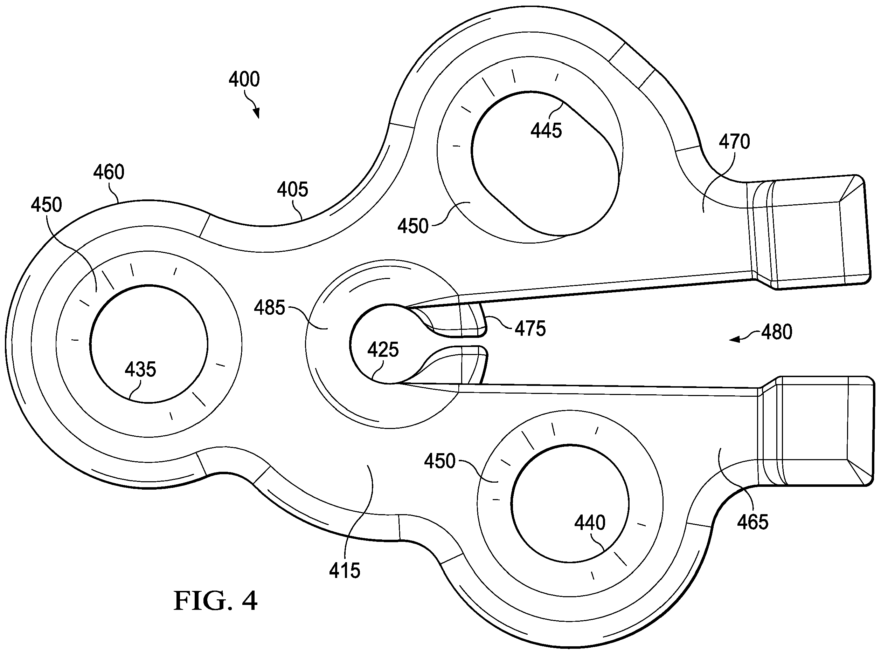

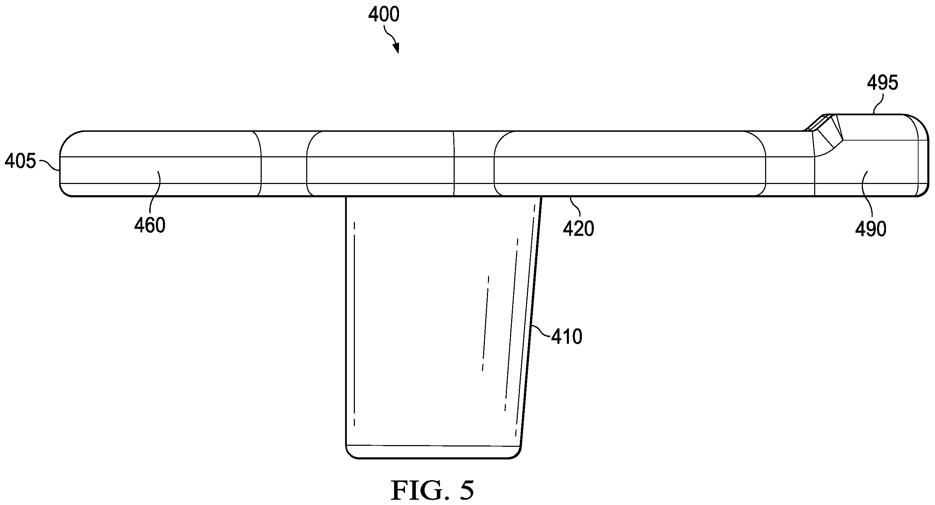

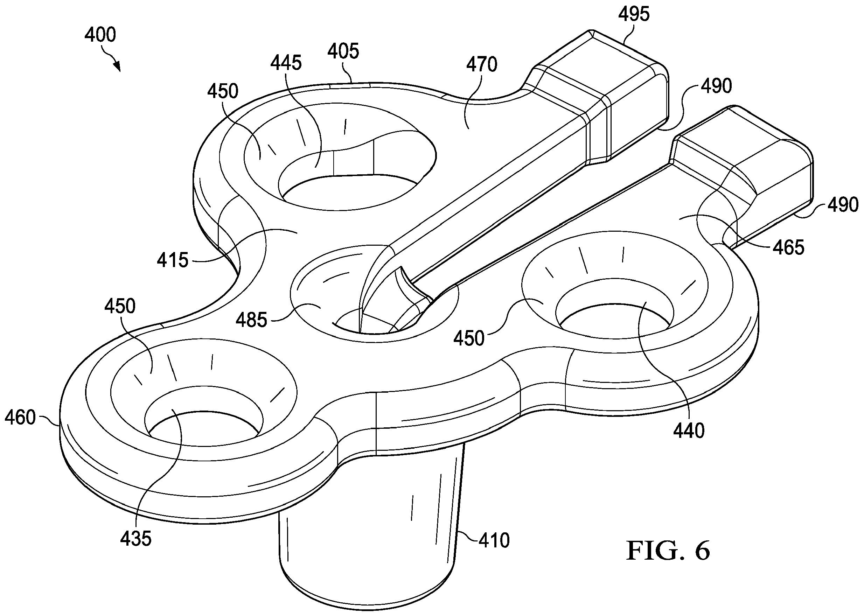

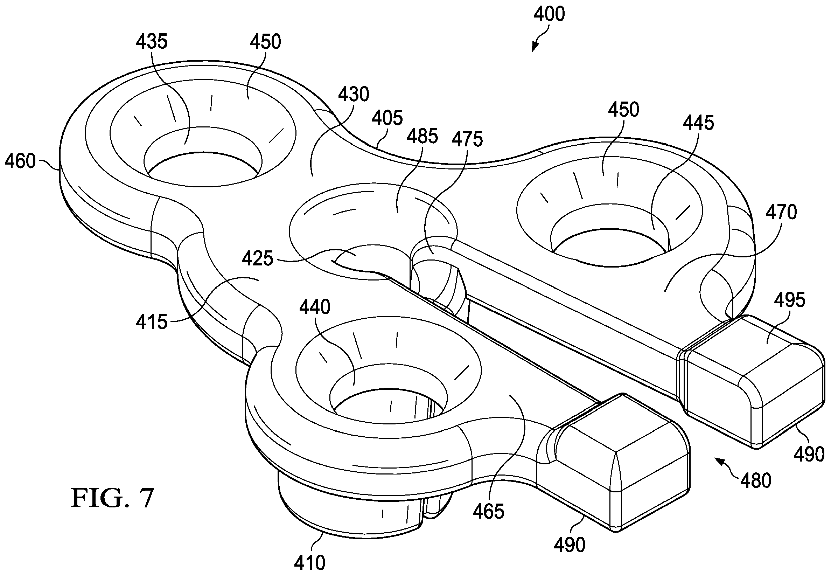

In accordance with various embodiments, referring now to Figures 4-8, the lead fixation device 400 comprises a mounting plate 405 and an insertion collar 410. The insertion collar 410 may be located at a central portion 415 of the mounting plate 405. The insertion collar 410 depends downwardly from a lower surface 420 of the mounting plate 405. The insertion collar 410 may be generally cylindrical and comprise an insertion hole 425 where a DBS lead 430 may be placed. In one embodiment, the insertion collar 410 may taper inwardly as it depends away from the lower surface 420. The insertion hole 425 may extend though to an upper surface of the mounting plate 405.

The mounting plate 405 may comprise a pair of mounting holes 435, 440 and a compression hole 445, which extend therethrough. The first and second mounting holes 435, 440 may be generally cylindrical. The first and second mounting holes 435, 440 may be fixed once installed. Each of the first and second mounting holes 435, 440 contains an inwardly beveled sidewall 450 that receives a countersunk head of a mounting screw 455. The compression hole 445 may comprise an elongate hole that allows for adjustment of the lead fixation device 400 during installation.

The first mounting hole 435 may be located on a flange 460 that extends from the central portion 415 of the mounting plate 405. The second mounting hole 440 and the compression hole 445 reside on a pair of arms 465, 470 that extend from the central portion 415 opposite of the first mounting hole 435. A space exists between the arms 465, 470 such that they can be pressed inwardly toward one another during installation. The arm 470 containing the compression hole 445 is rotatable with regard to the arm 465 containing the second mounting hole 440 such that when the two arms 465, 470 are pressed together, the space between the two arms 465, 470 creates a channel 480 to guide the DBS lead 430 exiting the patient/user.

The insertion hole 425 may comprise a cutout area 475 where a portion of a sidewall 485 of the insertion hole 425 is removed. The location of the cutout area 475 between the arms 465, 470 allows the arm 470 containing the compression hole 445 to move with regard to the arm 465 containing the second mounting hole 440. The arms 465, 470 each contain a finger flange 490 with a raised portion 495. The raised portions 495 allow the physician to press or pinch the arms 465, 470 toward one another during installation.

The lower surface 420 of the mounting plate 405 generally abuts the patient/user's skull and the insertion collar 410 may be may be inserted into the bone opening, burr hole or single twist drill opening of patient/user. The upper surface of the mounting plate 405 is generally flush with or sits on top of the patient/user's scalp. This configuration provides a generally invisible or inconspicuous lead fixation device.

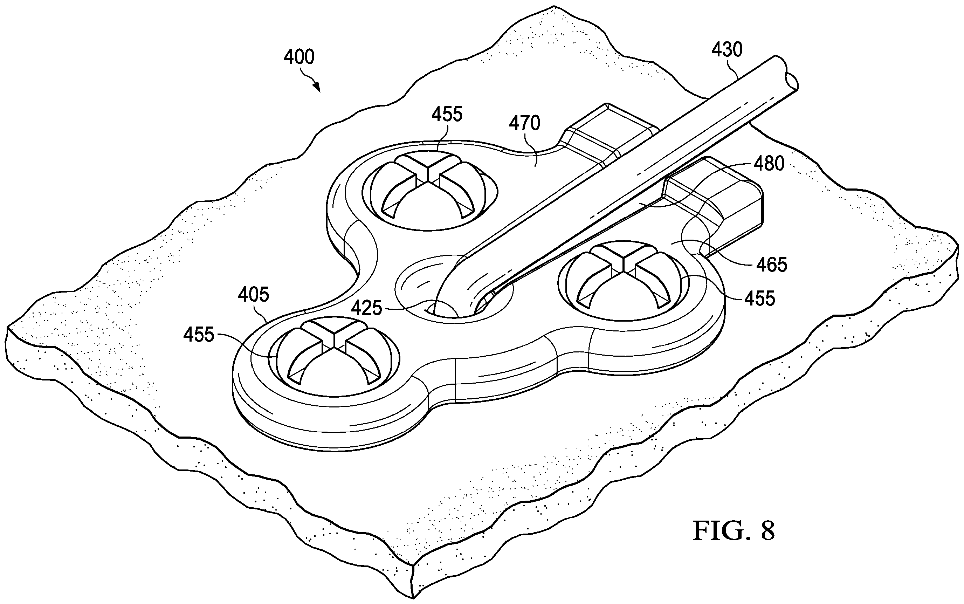

Figures 4-7 show the lead fixation device 400 in an open position while Figure 8 shows the lead fixation device 400 in an installed, closed position. In the closed position, the channel 480 formed within the space by the arms 465, 470 guides the DBS lead 430 that exits the user's skull in a desired direction.

In operation, the DBS lead 430 is inserted within a burr hole or single twist drill opening in the user/patient's skull. While the lead fixation device 400 in oriented in the open position, shown in Figures 4-7, the DBS lead 430 is placed within the insertion hole 425 located in the insertion collar 410. The doctor may then fasten the lead fixation device 400 to the skull by inserting screws 455 within the first and second mounting holes 435, 440. The DBS lead is then placed between the arms 465, 470. The doctor may then press or pinch the raised portions 495 of the arms 465, 450 toward one another and insert a screw 455 within the compression hole 445. The DBS lead 430 will now reside within the channel 480 formed within the space between the arms 465, 470.

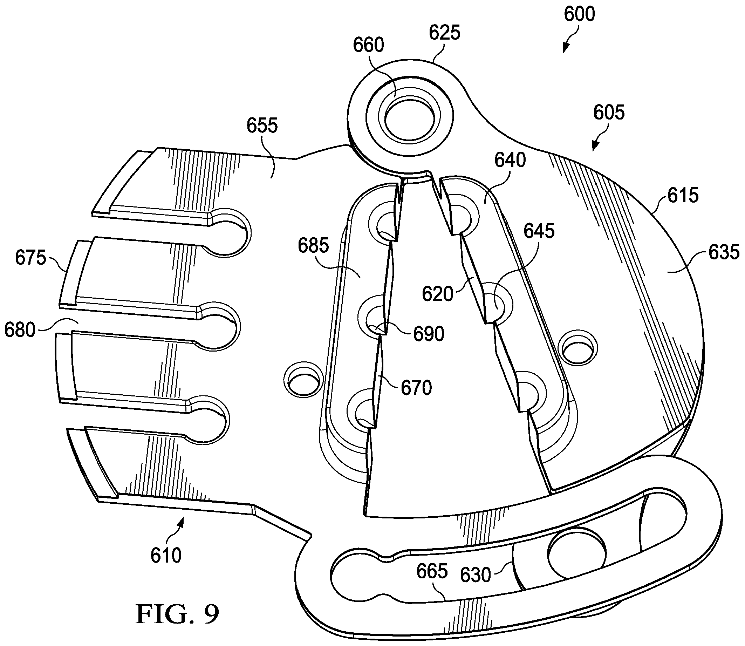

Referring now to Figures 9-11, in another embodiment, a low profile lead fixation device 600 is contemplated that is both faster and easier to use than any currently available system. The low profile lead fixation device 600 does not require any assembly or removal of the stereotactic at time of lead fixation. The low profile lead fixation device 600 may be on the order of a millimeter in thickness, produces no visible bump, and avoids the need for countersinking the fixation device by excessive skull drilling at the time of lead placement. In various embodiments low profile lead fixation device 600 may range from approximately 2mm to 0.5mm in thickness. In one embodiment, low profile lead fixation device 600 may be 1 mm in thickness. In one embodiment, low profile lead fixation device 600 may be less than 1 mm in thickness.

In accordance with various embodiments, referring now to Figures 9-11, the low profile lead fixation device 600 will be discussed. The low profile lead fixation device 600 comprises a mounting plate having a first arm 605 and a second arm 610. The first and second arms 605, 610 are rotatable and coupled to each other. In one embodiment the first and second arms 605, 610 are press-fit together at a rotating hinge site with a circular flange collar. In other words, one fitting inside in each arm resides within the other. The second arm 610 is rotatable with regard to the first arm 605 such that when the two arms are pressed together, the lead fixation device 600 may secure the DBS lead to the patient/user.

The first arm 605 may comprise a curved edge 615 and a substantially straight edge 620 located between a first flange 625 and a second flange 630. A lower surface 635 of the first arm 605 may comprise a raised protrusion 640 residing along the substantially straight edge 620. The raised protrusion 640 may comprise at least one arcuate groove 645. In one embodiment the raised protrusion 640 may comprise 3 arcuate grooves 645.

The second arm 610 may comprise an upper surface 650, a lower surface 655, a first flange 660, and a ring slot 665. The second arm 610 may comprise a substantially straight edge 670 and a curved edge 675 that reside between the first flange 660 and the ring slot 665. The second arm 610 may comprise at least one exit channel 680. In one embodiment, the second arm 610 may comprise 3 exit channels 680. In one embodiment, each of the exit channels 680 may comprise a semi-circular hole. The semi-circular holes at each exit channel 680 are provided to allow easy insertion of the DBS lead wire for subsequent press fitting into the exit channel 680 for a secondary friction based fixation. The exit channels 680 are oriented generally perpendicular to the substantially straight edge 670 and extend from the straight edge 670 towards the curved edge 675.

The lower surface 655 of the second arm 610 may comprise a raised protrusion 685 residing along the substantially straight edge 670. The raised protrusion 685 may comprise at least one arcuate groove 690. In one embodiment, the raised protrusion 685 may comprise 3 arcuate grooves 690.

The lower surfaces 635, 655 of the first and second arms 610, 615 generally abut the patient/user's skull while the upper surface 650 is flush with patient/user's scalp. This configuration provides a generally invisible or inconspicuous lead fixation device.

The first flange 625 of the first arm 605 is coupled to the first flange 660 of the second arm 610. The two arms 605, 610 may be press fit together at the circular rotating hinge located at the first flanges 625, 660. The coupling of the first flanges 625, 660 causes the second arm 610 to rotate with respect to the first arm 605. The second flange 630 of the first arm 605 is aligned with the ring slot 665. An inner wall 695 of the ring slot 665 abuts the curved edge 615 of the first arm 605 and slides along when the second arm 610 is rotated about the first arm 605. The ring slot 665 may comprise a pair of notches 700, which are designed to engage the screw when the lead fixation device 600 is attached to the user.

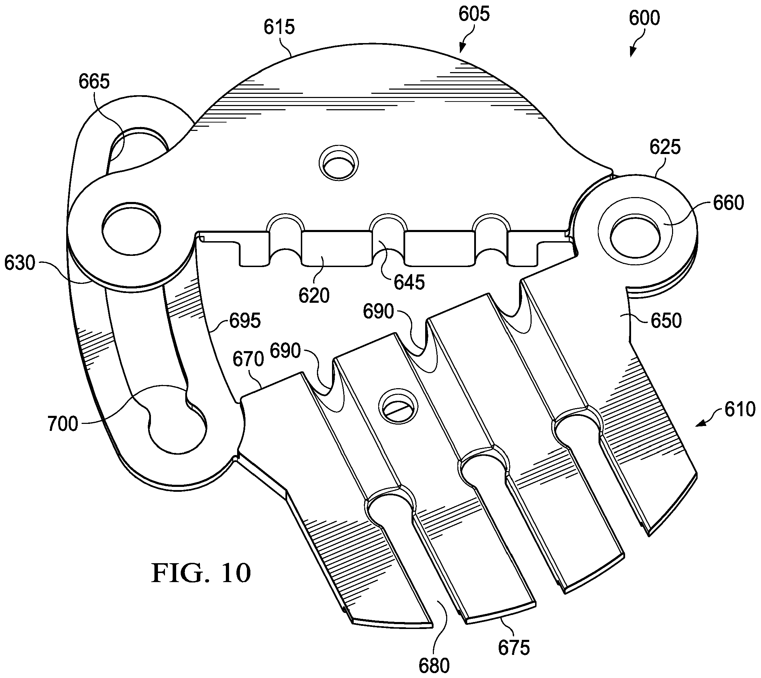

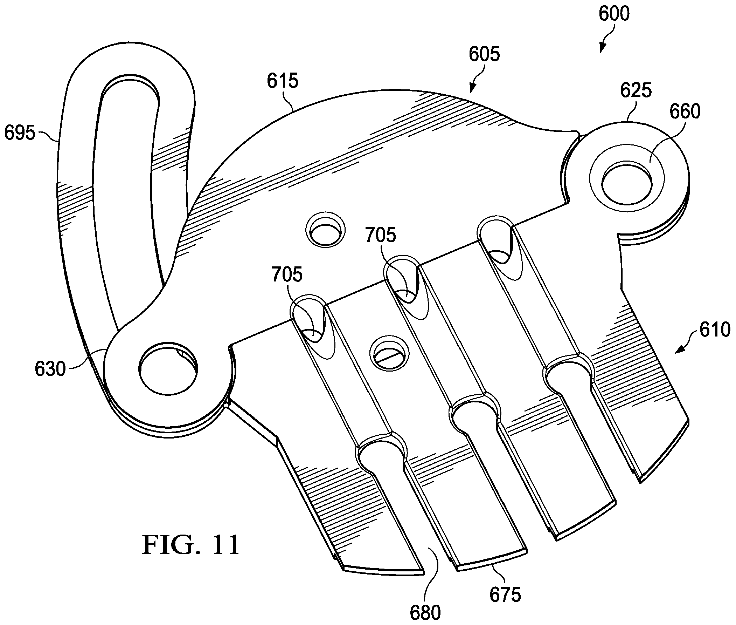

Figures 9 and 10 show the lead fixation device 600 in an open position while Figure 11 shows the lead fixation device 600 in a closed position. In the closed position, the raised protrusions 640, 685 abut and the arcuate grooves 645, 690 form at least one guide channel 705. In one embodiment 3 guide channels 705 are formed. The guide channels 705 receive the DBS lead that exits the user's skill.

In operation, the DBS lead is inserted within a burr hole in the user/patient's skull. While the lead fixation device 600 in oriented in the open position, shown in Figures 9 and 10, the DBS lead is placed within the arcuate groove 645 located in the raised protrusion 640 of the first arm 605. The second arm 610 is then rotated to the closed position, shown in Figure 3, where the arcuate groove 690 located in the raised protrusion 685 of the second arm 610 forms the guide channel 705 and clamps the DBS lead to hold it in place. A stylet within the DBS lead may then be removed and the DBS lead may be pressed to fold over and fit within the exit channel 680 for a second point of fixation for added security. The doctor may then fasten the lead fixation device 600 to the skull by inserting screws within the first flanged connection 625, 660 and the second flange 630 and ring slot 665 connection,

These and other embodiments for a lead fixation device may incorporate concepts, embodiments, and configurations as described above. The particular implementations shown and described are illustrative of the technology and its best mode and are not intended to otherwise limit the scope of the present technology in any way. Indeed, for the sake of brevity, conventional manufacturing, connection, preparation, and other functional aspects of the system may not be described in detail. Furthermore, the connecting lines shown in the various figures are intended to represent exemplary functional relationships and/or physical couplings between the various elements. Many alternative or additional functional relationships or physical connections may be present in a practical system.

The technology has been described with reference to specific exemplary embodiments. Various modifications and changes, however, may be made without departing from the scope of the present technology. The description and figures are to be regarded in an illustrative manner, rather than a restrictive one and all such modifications are intended to be included within the scope of the present technology. Additionally, the components and/or elements recited in any apparatus embodiment may be assembled or otherwise operationally configured in a variety of permutations to produce substantially the same result as the present technology and are accordingly not limited to the specific configuration recited in the specific examples. Benefits, other advantages and solutions to problems have been described above with regard to particular embodiments; however, any benefit, advantage, solution to problems or any element that may cause any particular benefit, advantage or solution to occur or to become more pronounced are not to be construed as critical, required or essential features or components.

As used herein, the terms "comprises", "comprising", or any variation thereof, are intended to reference a non-exclusive inclusion, such that an apparatus that comprises a list of elements does not include only those elements recited, but may also include other elements not expressly listed or inherent to such apparatus. Other combinations and/or modifications of the above-described structures, arrangements, applications, proportions, elements, materials or components used in the practice of the present technology, in addition to those not specifically recited, may be varied or otherwise particularly adapted to specific environments, manufacturing specifications, design parameters or other operating requirements without departing from the general principles of the same.

The present technology has been described above with reference to an exemplary embodiment. However, changes and modifications may be made to the exemplary embodiment without departing from the scope of the present technology. These and other changes or modifications are intended to be included within the scope of the present technology, as expressed in the following claims.

## Claims

1. A lead fixation device (100) for attaching a deep brain stimulation lead within in a burr hole in a human skull, the lead fixation device (100) being movable from an open position to a closed position, comprising:
a mounting plate (105) comprising:
a first arm (110) comprising:
a first flange (120),
a first mounting hole (135), and
a first inner wall (150);
a second arm (115) comprising:
a second flange (125) coupled to the first flange at a connection point,
a second mounting hole (140), and
a second inner wall (155);
wherein the first arm (110) is rotatably coupled to the second arm (115) at the connection point between the first flange (120) and the second flange (125), **characterised in that** the first mounting hole (135) and the second mounting hole (140) do not overlap with any mounting hole when the lead fixation device (100) is in the closed position.

2. The lead fixation device (100) of claim 1, comprising:
a first arcuate groove (170) located on the first inner wall (150) of the first arm (110), and
a second arcuate groove (175) located on the second inner wall (155) of the second arm (115).

3. The lead fixation device (100) of claim 2, wherein the first (170) and second (175) arcuate grooves form a guide channel to receive the lead when the lead fixation device (100) is in a closed position.

4. The lead fixation device (100) of claim 3, wherein the first (135) and second (140) mounting holes are located outwardly of the guide channel.

5. The lead fixation device (100) of any one of the preceding claims, wherein the first (150) and second (155) inner walls form an exit channel to receive the lead when the lead fixation device (100) is in a closed position.

6. The lead fixation device (100) of any one of the preceding claims, wherein the first flange (120) is rotatably coupled to the second flange (125).

7. The lead fixation device (100) of any one of the preceding claims, wherein:
the first mounting hole (135) is located at approximately a midpoint of the first arm (110), and
the second mounting hole (140) is located at approximately a midpoint of the second arm (115).

8. The lead fixation device (100) of any one of the preceding claims, further comprising a locking mechanism (270) comprising:
a first finger (275) having a raised portion (285) and depending from an upper surface of the first flange (120), and
a second finger (280) having a raised portion (300) depending from an upper surface of the second flange (125);
wherein the first (285) and second (300) raised portions are configured to lock the lead fixation device (100) in a closed position.

9. The lead fixation device (100) of any one of the preceding claims, wherein each of the first (150) and second (155) inner walls comprise a curved portion, which are oriented parallel to one another.

10. The lead fixation device (100) of any one of the preceding claims, wherein the first flange (120) is rotatably coupled to the second flange (125).

## Patentansprüche

1. Leitungsfixierungsvorrichtung (100) zum Anbringen einer Leitung für die tiefe Gehirnstimulation innerhalb eines Bohrlochs in einem menschlichen Schädel, wobei die Leitungsfixierungsvorrichtung (100) von einer offenen Position in eine geschlossene Position bewegbar ist, umfassend:
eine Befestigungsplatte (105), umfassend:
einen ersten Arm (110), umfassend:
einen ersten Flansch (120),
ein erstes Befestigungsloch (135) und
eine erste Innenwand (150);
einen zweiten Arm (115), umfassend:
einen zweiten Flansch (125), der mit dem ersten Flansch an einem Verbindungspunkt gekoppelt ist,
ein zweites Befestigungsloch (140), und
eine zweite Innenwand (155);
wobei der erste Arm (110) mit dem zweiten Arm (115) an dem Verbindungspunkt zwischen dem ersten Flansch (120) und dem zweiten Flansch (125) drehbar gekoppelt ist, **dadurch gekennzeichnet, dass** das erste Befestigungsloch (135) und das zweite Befestigungsloch (140) sich nicht mit einem beliebigen Befestigungsloch überlappen, wenn die Leitungsfixierungsvorrichtung (100) in der geschlossenen Position ist.

2. Leitungsfixierungsvorrichtung (100) nach Anspruch 1, umfassend:
eine erste bogenförmige Rille (170), die sich an der ersten Innenwand (150) des ersten Arms (110) befindet, und
eine zweite bogenförmige Rille (175), die sich an der zweiten Innenwand (155) des zweiten Arms (115) befindet.

3. Leitungsfixierungsvorrichtung (100) nach Anspruch 2, wobei die erste (170) und die zweite (175) bogenförmige Rillen einen Führungskanal ausbilden, um die Leitung aufzunehmen, wenn die Leitungsfixierungsvorrichtung (100) in einer geschlossenen Position ist.

4. Leitungsfixierungsvorrichtung (100) nach Anspruch 3, wobei sich das erste (135) und das zweite (140) Befestigungsloch außerhalb des Führungskanals befinden.

5. Leitungsfixierungsvorrichtung (100) nach einem der vorstehenden Ansprüche, wobei die erste (150) und die zweite (155) Innenwand einen Austrittskanal ausbilden, um die Leitung aufzunehmen, wenn die Leitungsfixierungsvorrichtung (100) in einer geschlossenen Position ist.

6. Leitungsfixierungsvorrichtung (100) nach einem der vorstehenden Ansprüche, wobei der erste Flansch (120) mit dem zweiten Flansch (125) drehbar gekoppelt ist.

7. Leitungsfixierungsvorrichtung (100) nach einem der vorstehenden Ansprüche, wobei:
sich das erste Befestigungsloch (135) in etwa an einem Mittelpunkt des ersten Arms (110) befindet, und
sich das zweite Befestigungsloch (140) in etwa an einem Mittelpunkt des zweiten Arms (115) befindet.

8. Leitungsfixierungsvorrichtung (100) nach einem der vorstehenden Ansprüche, ferner umfassend einen Verriegelungsmechanismus (270), umfassend:
einen ersten Finger (275), der einen erhöhten Abschnitt (285) und abhängend von einer oberen Oberfläche des ersten Flansches (120) aufweist, und
einen zweiten Finger (280), der einen erhöhten Abschnitt (300) abhängend von einer oberen Oberfläche des zweiten Flansches (125) aufweist;
wobei der erste (285) und der zweite (300) erhöhte Abschnitt konfiguriert sind, um die Leitungsfixierungsvorrichtung (100) in einer geschlossenen Position zu verriegeln.

9. Leitungsfixierungsvorrichtung (100) nach einem der vorstehenden Ansprüche, wobei jede der ersten (150) und der zweiten (155) Innenwand einen gekrümmten Abschnitt umfassen, die parallel zueinander ausgerichtet sind.

10. Leitungsfixierungsvorrichtung (100) nach einem der vorstehenden Ansprüche, wobei der erste Flansch (120) mit dem zweiten Flansch (125) drehbar gekoppelt ist.

## Revendications

1. Dispositif de fixation de sonde (100) permettant de fixer une sonde de stimulation cérébrale profonde à l'intérieur d'un trou de trépan dans un crâne humain, le dispositif de fixation de sonde (100) étant mobile d'une position ouverte à une position fermée, comprenant :
une plaque de montage (105) comprenant :
un premier bras (110) comprenant :
une première bride (120),
un premier trou de montage (135), et
une première paroi interne (150) ;
un second bras (115) comprenant :
une seconde bride (125) accouplée à la première bride au niveau d'un point de liaison,
un second trou de montage (140), et
une seconde paroi interne (155) ;
dans lequel le premier bras (110) est accouplé de manière rotative au second bras (115) au niveau du point de liaison entre la première bride (120) et la seconde bride (125), **caractérisé en ce que** le premier trou de montage (135) et le second trou de montage (140) ne se chevauchent pas avec un quelconque trou de montage lorsque le dispositif de fixation de sonde (100) est en position fermée.

2. Dispositif de fixation de sonde (100) selon la revendication 1, comprenant :
une première rainure arquée (170) située sur la première paroi interne (150) du premier bras (110), et
une seconde rainure arquée (175) située sur la seconde paroi interne (155) du second bras (115).

3. Dispositif de fixation de sonde (100) selon la revendication 2, dans lequel les première (170) et seconde (175) rainures arquées forment un canal de guidage pour recevoir la sonde lorsque le dispositif de fixation de sonde (100) est en position fermée.

4. Dispositif de fixation de sonde (100) selon la revendication 3, dans lequel les premier (135) et second (140) trous de montage sont situés vers l'extérieur du canal de guidage.

5. Dispositif de fixation de sonde (100) selon l'une quelconque des revendications précédentes, dans lequel les première (150) et seconde (155) parois internes forment un canal de sortie pour recevoir la sonde lorsque le dispositif de fixation de sonde (100) est en position fermée.

6. Dispositif de fixation de sonde (100) selon l'une quelconque des revendications précédentes, dans lequel la première bride (120) est accouplée de manière rotative à la seconde bride (125).

7. Dispositif de fixation de sonde (100) selon l'une quelconque des revendications précédentes, dans lequel :
le premier trou de montage (135) est situé à approximativement un point médian du premier bras (110), et
le second trou de montage (140) est situé à approximativement un point médian du second bras (115).

8. Dispositif de fixation de sonde (100) selon l'une quelconque des revendications précédentes, comprenant en outre un mécanisme de verrouillage (270) comprenant :
un premier doigt (275) ayant une partie surélevée (285) et dépendant d'une surface supérieure de la première bride (120), et
un second doigt (280) ayant une partie surélevée (300) dépendant d'une surface supérieure de la seconde bride (125) ;
dans lequel les première (285) et seconde (300) parties surélevées sont conçues pour verrouiller le dispositif de fixation de sonde (100) en position fermée.

9. Dispositif de fixation de sonde (100) selon l'une quelconque des revendications précédentes, dans lequel chacune des première (150) et seconde (155) parois internes comprend une partie incurvée, qui sont orientées parallèlement l'une à l'autre.

10. Dispositif de fixation de sonde (100) selon l'une quelconque des revendications précédentes, dans lequel la première bride (120) est accouplée de manière rotative à la seconde bride (125).
